# EUROPEAN PATENT SPECIFICATION

(11) **EP 3 727 271 B1**
(45) Date of publication and mention of the grant of the patent: **20.09.2023**
(21) Application number: 18839874.7
(22) Date of filing: 20.12.2018
(51) Int. Cl.: A61H 3/00, A61H 1/02

(54) **WEARABLE ROBOT WITH PERFECTED CONTROL ARCHITECTURE**
WEARABLE-ROBOTER MIT PERFEKTIONIERTER STEUERUNGSARCHITEKTUR
ROBOT POUVANT ÊTRE PORTÉ AYANT UNE ARCHITECTURE DE COMMANDE PERFECTIONNÉE

(30) Priority: 21.12.2017 IT 201700148580
(43) Date of publication of application: 28.10.2020
(73) Proprietor: Scuola Superiore di Studi Universitari e di Perfezionamento Sant'Anna, 56127 Pisa (IT)
(72) Inventor: VITIELLO, Nicola, 56025 Pontedera (PI) (IT); CIANCHETTI, Matteo, 06131 Perugia (IT); PARRI, Andrea, 57021 Campiglia Marittima (LI) (IT); GRASSI, Angela, 5616BA Eindhoven (NL); MARTINI, Elena, 50124 Firenze (IT); CREA, Simona, 55100 Lucca (IT)
(74) Representative: Pietri, Simona
(86) International application number: PCT/IB2018/060441
(87) International publication number: WO 2019/123374

(56) References cited:
- EP-A1- 2 832 338
- WO-A2-2008/094191
- US-A1- 2015 134 080
- US-A1- 2016 113 831
- US-A1- 2016 331 560
- US-B2- 6 497 672

## Description

The present invention belongs to the field of wearable robotic devices, and in particular relates to a wearable robot that comprises improved means for measuring at least one movement variable of an articulation assisted by such robot, in order to elaborate an assistive control strategy of a motorized module of said robot such as a joint that corresponds to such articulation.

A particularly significant problem in the field of wearable robotic devices is that of developing a suitable assistive strategy, i.e. suitably regulating the transfer of mechanical power from the device to the user. The aim is to obtain a system that provides assistance consistently with the user's articular movement, adapting to voluntary variations (e.g. the rhythm of the step) and those dictated by the external environment (e.g. the slope of the ground where the user walks). Clearly, the system sets out to dispense energy to the user in appropriate phases of the movement with suitable intensity for the specific and momentary requirements, adapting its action to the user's movement variations.

To obtain such adaptability in real time it is necessary to have one or more sensors adapted to monitor in real time the input variables of the control algorithm of the exoskeleton device and an adaptive assistive strategy able to update the action of the robot based on the variables measured by the sensors; an appropriate mechanical structure is also necessary in order to allow the effective transfer of mechanical power and a reliable measurement of the input variables.

Adaptive walking assistive strategies may be based on the acquisition of muscular activation signals or on the detection of relevant events of the step cycle such as, for example, the contact of the foot with the ground. The detection of the event provides a reference for starting a new phase of the step on which to define an assistive profile that depends on the phase of the cyclic movement but not on the specific articular kinematics. An example of an assistive strategy is presented in the work by Ronsse et al. entitled "Oscillator-based assistance of cyclical movements: model-based and model-free approaches*";* in this case an assistive torque is generated dependent not only on the phase of the step but also on the specific angular trajectory of the articulation. This strategy was tested on an exoskeleton of a lower limb known as LOPES (see previously quoted article) and in the article by Giovacchini et al. dated 2015 entitled "A light-weight active orthosis for hip movement assistance" and was applied to an exoskeleton for assisting the flexion-extension movements of the hip.

Such known assistive strategy calculates the reference torque to be delivered for the specific articulation based on the angular movement of the articulation itself using angular sensors such as joint encoders integrated into the transmission chain of the exoskeleton robot. Such sensors produce a measurement of the articular angle that can be considered reliable as long as there is a perfect coupling between the joint of the robot and that of the articulation. In the real case, the robot, which is rigid, interfaces with the body segment comprising soft and therefore yielding tissues (such as muscular tissue and fatty tissue), therefore causing a viscoelastic type coupling. The transfer of mechanical power to the user therefore generates discrepancy between the measurement of the angle of the robot and the actual angle of the human articulation with which the robot is interfaced, due to the non-ideal coupling between the robot and the user. In fact, the robotic joint will measure a different angle from that of the articulation, resulting from the sum of two components, i.e. the real angular position of the user's articulation and the contribution provided by the compression of the soft tissue in response to the mechanical action of the rigid transmission means at the human-robot interface. Such error in the angular measurement triggers an unstable interaction as the consequent planning of the assistive torque is based on a measurement affected by the error introduced by the assistive action itself.

Consequently, the energy transferred could even disturb the movement, establishing a mechanism of propagation and amplification of the error which leads to instability. This phenomenon is even more evident the higher the assistive power to be transferred (d'Elia et al. 2017). The performance of the device is therefore limited within a maximum intensity of the assistive power. In particular, based on the experimental activity performed, it is estimated that the maximum assistive power to be transferred is limited to around 25-30% of the physiological torque of the human articulation according to scientific literature (about 10 Nm for the hip joint in a healthy subject weighing 70 kg- *Winter, 2009*)*.*

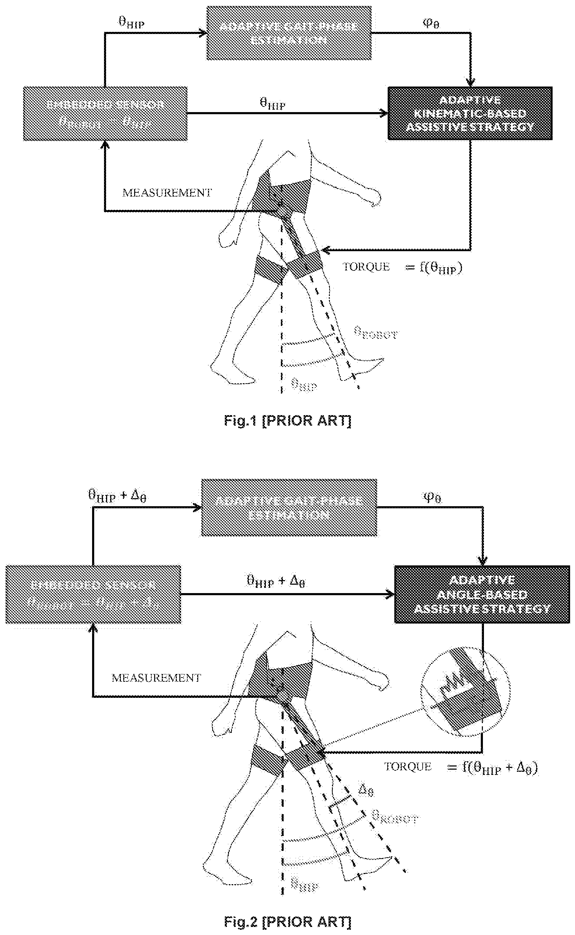

In summary, and taking the example of an exoskeleton that assists the hip movements of a user, if in the ideal case (Figure 1) the torque delivered to the hip joint is a function of the flexion-extension angle of the articulation (in this case the hip), in the real case such torque is delivered as a function of a measured angle that does not correspond to the flexion-extension angle of the hip but diverges therefrom by an error value Δθ, actually a consequence of the error in the measurement generated by the transfer of mechanical power due to the visco-elastic coupling between the robot and the limb (Figure 2).

As well as visco-elastic interactions and resulting compressions of the soft tissues, the human-robot interface can be affected by a misalignment of the rotation axes of the robotic joint and the human joint, or by relative slipping phenomena between the robot and the body segment. All these phenomena lead to an incorrect measurement of the angle of rotation of the articulation. For these reasons, in many cases alternative solutions need to be found, for example by dispensing predefined assistive actions that are scaled based on the estimate of the assisted movement phase that do not consider the specific angular trajectory of the articulation.

For example, in some examples of known robots, sensor means are used to perform kinematic measurements on the user's body, limited to speeds or accelerations used solely for extrapolating an estimate of the user's movement phase through appropriately designed algorithms. The process of estimating the phase of the step cycle often requires complex computational techniques that cannot always be actuated through kinematic sensor means. Moreover, in literature, means such as plantar pressure sensors are preferred therefore adding a further element to the mechatronic architecture of the wearable robot. Once the phase of the movement has been estimated, the assistive torque can be generated: (i) by interpolating a predefined curve on the phase reference, (ii) through a function that generates the assistive torque from a parametric equation of the phase, (iii) by using complex models whose characteristics vary from one user to another and that may require heavy online processing operations of the performed measurements (e.g. Kalman filter) that can limit the usability thereof.

Examples of this type of robot are described in patent publications US2015/134080 or US2016113831.

Document US2015/134080 A1 discloses a wearable robot according to the preamble of claim 1.

The object of the present invention is therefore that of providing a wearable robotic device that allows the movement of a person wearing it to be effectively assisted, based on the angular trajectory itself of the assisted joint also for high torque values.

In particular, it is an object of the invention to implement, in a wearable robotic device, improved means for measuring at least one movement variable of the articulation with which said device is associated; it is in particular an objective of the invention that such improved measurement means provide a reliable measurement based on which to implement an assistive strategy of the robotic device itself.

These and other objectives are reached by the wearable robotic device according to the invention, wherein the wearable robot comprises:
- at least one motorized module adapted to be arranged at a limb of the user, the module comprising a mobile joint adapted to be associated to an articulation of the limb; the mobile joint being mobile according to at least one degree of freedom associated with a movement of the articulation.

Wherein the robotic device module comprises a first kinematic chain comprising, in addition to the mobile joint:
- actuation means for driving or supporting the movement of the joint according to the at least one degree of freedom;
- transmission means interposed between the actuating means and the joint;

The robotic device further comprising:
- energy storage and release means, suitable for the activation of the actuation means;
- sensor means arranged at the articulation and suitable for detecting a detected value of the at least one parameter of the respective movement of the articulation, wherein the sensor means are also kinematically uncoupled from the first kinematic chain;
- a control unit designed to control the first kinematic chain on the basis of an assistive control strategy of the wearable robot; the control strategy being elaborated as a function of the value of the motion parameter detected by the sensor means.

The concept of kinematic uncoupling is to be understood in the sense that the sensory system is entrusted to means or to a device that are kinematically independent from the kinematic chain appointed for the transmission of assistive power through the motorized module and that operate on the user, directly or indirectly, without functional interaction with the aforesaid chain.

The characteristics and advantages of the wearable robot according to the present invention will appear more clearly from the following description of an embodiment thereof, provided by way of non-limiting example with reference to the appended drawings wherein:
- Figure 1 schematically represents an ideal example of the control of an exoskeleton robot, wherein the torque dispensed to a mobile articulation joint is a function of the value of a predefined movement variable of the limb with which such robot is associated, where the value of such movement variable corresponds exactly to the value of a corresponding movement variable of such joint;
- Figure 2 instead represents a real case applied to the example of Figure 1, where the value of the predefined movement variable of the corresponding joint differs from the value of the movement variable of the articulation of an error Δθ;
- Figure 3 is a conceptual diagram of the solution according to the invention;
- Figures 4a and 4b schematically represent a first embodiment of the invention;
- Figure 5 represents a second embodiment of the invention;
- Figure 5a is an enlargement of a portion of the robot of Figure 5;
- Figure 6 represents a third embodiment of the invention; and
- Figure 6a is an enlargement of a portion of the robot of Figure 6.

With reference to the aforementioned figures, the robotic device 1 according to the invention is adapted for being worn by a user in order to assist a limb 2 and comprises at least one motorized module 10 suitable for being associated or corresponding kinematically with an articulation 20 of the assisted limb 2. By way of example, therefore, reference is made for simplicity to a hip orthosis.

The motorized module 10 has at least one degree of freedom at a mobile joint 100 adapted for corresponding with the articulation, said degree of freedom identified with the measurment of the value of at least one movement parameter.

In order to move the motorized module in cooperation with the movement of the limb 2 actuation means 11 are provided, shown schematically in the figures, that insist on a transmission joint 101 of the robot and, through kinematic transmission means 12, also portrayed schematically, transmit mechanical power to the articulation 20, through the mobile joint 100. In more detail, the kinematic transmission means comprise a first segment 12' that extends between the actuation means 11 and the joint 100 and a second segment 12" that extends between the joint 100 and an anchorage point 21 to the limb 2 of the motorized module 10.

The assembly of these components, or actuation means 11, transmission means 12 and mobile joint (100) represents a first power transmission kinematic chain. In turn, the actuation means are operatively connected to energy storage and release means 13 such as a battery for example. The control of the movement mobile joint 100 is implemented by a control unit 14 that elaborates an assistive strategy, and has the purpose of adaptively controlling the dispensing of a torque force to the mobile joint through the activation of the actuation means; in particular, assistive strategy means an actuation strategy of the joint so that it provides the limb a torque such as to support its residual movement.

Such assistive strategy is elaborated on the basis of the at least one movement reference value mentioned above; this value is detected by sensor means 3 that, according to a peculiar aspect of the invention, are uncoupled from the kinematic transmission means of the joint. It is therefore to be understood that the sensor means are not integrated in the transmission chain of the robot, i.e. they are operatively unconstrained from the actuation means and not controlled thereby.

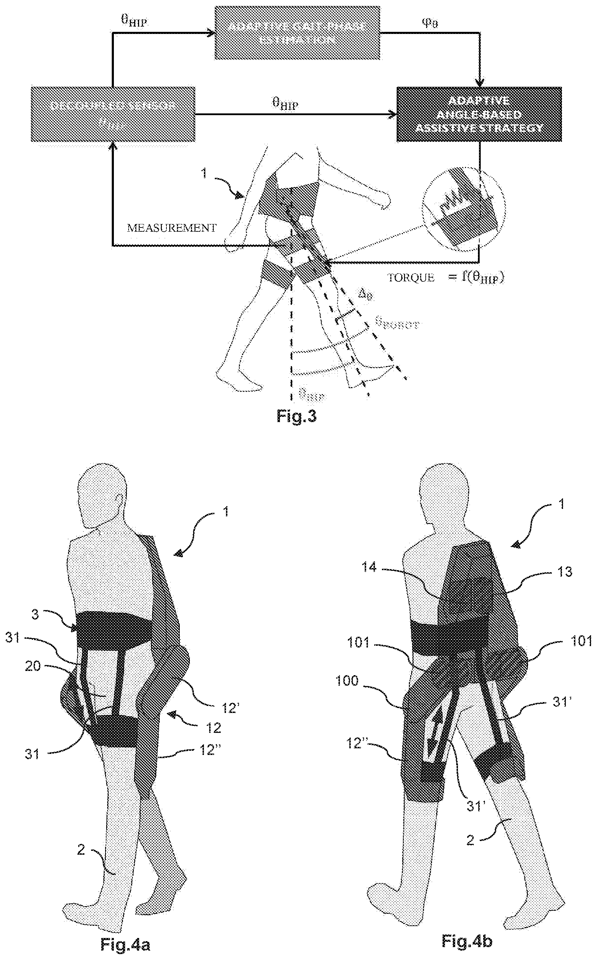

With particular reference to the example of Figure 3, the motorized module 10 is as mentioned associated with the articulation 20 of the hip 2, therefore acts in association therewith to support and/or assist the flexion-extension movement of the hip itself. The reference value of such movement is therefore the value of the flexion-extension angle of the hip *θ_{HIP}*. As the sensor means are uncoupled from said transmission chain of the robot, the reference value detected thereby corresponds to the effective value of the reference parameter of the movement of the articulation. The reference value detected by the sensor means 3 is then used by the control means as a parameter in the control strategy assistive of the robot, which controls the actuation means so that they supply to the mobile joint 100 a torque C=f(*θ_{HIP}*) as a function of such flexion-extension angle detected.

In an embodiment shown in Figure 4a and in Figure 4b, the sensor means 3 can comprise elastic means directly associated with the limb so that a movement of the limb itself corresponds to a deformation of such elastic means; the reference parameter value detected is a function of such deformation value of the elastic means.

Preferably the elastic means are subject to a one-directional type deformation. Such one-directional deformation is preferably performed according to a length extension direction of the limb 2.

For example, the elastic means may provide at least one band of elasticized and conductive engineered fabric 31. The at least one band has a prevalent extension according to a preferred extension direction and is placed in association with the limb according to the direction of the length of the limb itself.

Again, the at least one band is associated with the limb so as to be pre-tensioned with respect to its length at rest; therefore, it undergoes an elongation deformation in the preferred extension direction thereof consistent with the movement of the limb involving the articulation and by effect thereof. The angle of rotation of the articulation is therefore evaluated as a function of the deformation of the at least one band, or as a function of its electrical conductivity variation due to the aforesaid elongation, expressed by a legible signal for obtaining the value of the movement parameter of the articulation.

Possibly, the elastic means may comprise two bands of fabric 31, 31' and in this case the value of the parameter relative to the movement of the limb may be measured as a function of the detected values of such parameter based on signals obtained from both, appointed to detect the flexion movement and the extension movement of the limb, respectively.

The bands can advantageously, for that purpose, be placed in association with respective portions of the limb involving said articulation, opposite each other according to a flexion-extension plane of the articulation itself so that as a function of the flexion or extension movement of the limb itself, one of the two is subject to elongation, while the other remains unloaded and not taut. In detail, during a flexion-extension movement of the limb, the angle of rotation of the articulation 20 will therefore be calculated as a sum of the angles of flexion and extension, each measured as a function of the conductibility variation of a respective band. The affixing of the bands may be secured with a garment or wearable harness 32, or even with adhesive means.

Figures 4a and 4b specifically relate to a wearable robot with two robotized modules for the hips. For each module a first band 31 is placed on the front of the limb 2 and slides from the abdomen to the proximal segment of the lower limb (as can be seen in Figure 4a); a second band 31' (visible in Figure 4b) is placed on the back of the limb A and slides from the rear part of the back at the pelvis to the rear part of the proximal segment of the lower limb. During the step, the articulation cyclically performs a flexion movement (positive angle of rotation of the articulation 20 with reference to a reference axis perpendicular to the floor that crosses the articulation itself) (Figure 4a) and an extension movement (negative angle of rotation of the articulation 20, still in relation to the same reference axis) (Figure 4b). The extension movement has a deformation effect on the first band of engineered fabric 31, i.e. the one placed on the front part thereof. The flexion movement instead has a deformation effect on the second band 31', i.e. the one placed on the rear of the limb itself. The desired articulation angle is therefore the sum of the angles of extension and flexion evaluated as a function of the conductivity variation value of the first band 31 and the second band 31' respectively.

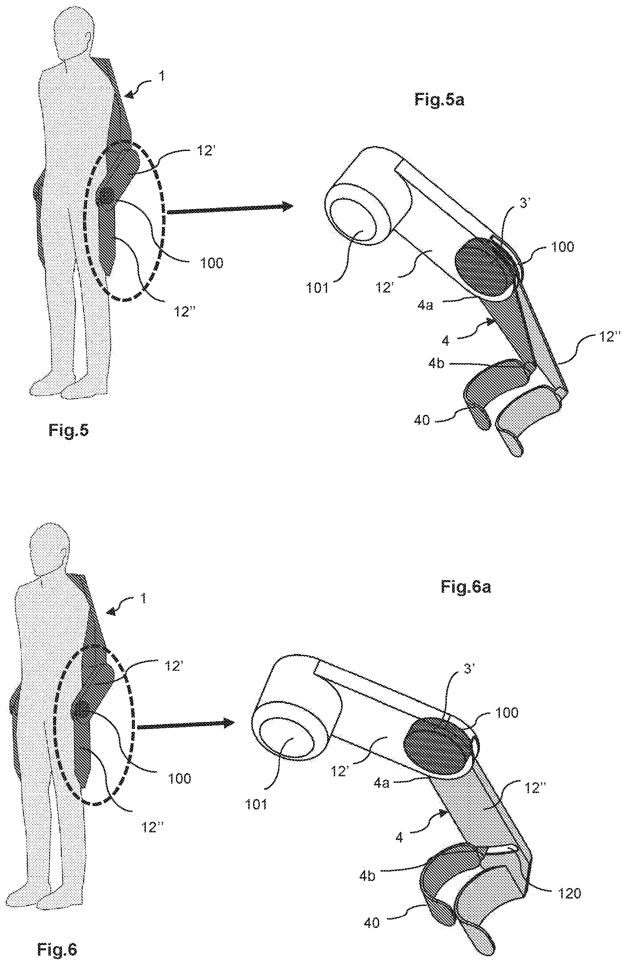

Now with reference to figures 5 to 6a a further embodiment is shown where the sensor means 2' comprise:
- a sensor 3' adapted to detect the value of the movement parameter directly on the articulation 20;
- a second kinematic chain 4 that integrates the sensor 3' and is uncoupled from the first transmission chain, being parallel thereto and interfaced with the articulation 20 through an axis of rotation parallel and/or coinciding with the axis of rotation of the articulation.

The second kinematic chain 4 has the function of maintaining the sensor in a suitable position for obtaining the measurement, i.e. in a proximal position to the articulation and therefore to the mobile joint. At the same time, the second kinematic chain, being independent from the transmission chain of the robot, determines the uncoupling between the sensor and the transmission chain.

Specifically, the second kinematic chain is materialized with a brace 4 that can be worn on the limb 2. The brace 4 extends between a proximal end 4a associated, as can be seen below, with the mobile joint 100 and a distal end 4b that mounts fixing means 40 (such as a band or the like) on the limb.

The brace 4 is constrained through its proximal end 4a freely to the mobile joint 100, i.e. it is not mechanically or operatively connected to the transmission chain 12, therefore it passively follows the movement of the limb.

Again, in a possible embodiment, the sensor is an angular type sensor that detects a value of the angle of rotation of the assisted articulation.

Examples of angular sensors 3' that can satisfy the measurement requirements just described may be chosen from one or more of those listed below by way of example, notwithstanding the fact the list is not to be considered exhaustive: - optical encoders; - magnetic sensors (such as Hall-effect angular position sensors).

The brace 4 extends parallel to the second segment 12" of the transmission chain, both in a more proximal position to the limb, or in a position interposed between the limb and the segment 12" of the first transmission chain (Figures 5 and 5a), and in a more distant position from the limb, i.e. in this case it is the segment 12" of the first transmission chain 12 (Figures 6 and 6a) interposed between the limb 2 and the brace 4. In this specific case a slot 120 is provided on the lower segment 12" to allow the free passage of the distal end 4b, for the connection of the brace to the limb through the fixing means 40. The brace is therefore free to move independently with respect to the first transmission chain and to follow the movement of the limb, thanks to the uncoupling guaranteed by the slot 120, within which the distal end 4b is free to move.

Those described above are just some examples of sensor means applicable to the robot according to the invention. Clearly other solutions may be provided, both in the form of sensor means applied directly to the user, and sensor means connected through kinematic chains uncoupled from the robot. On this point, the positioning of the electrogoniometers on the articulation can take place directly on the person.

The wearable robot according to the invention has various advantages. In particular, the uncoupling between the sensor means and at least the power transmission chain of the robot guarantees an accurate and real measurement of the value of the reference parameter of the movement of the assisted articulation, a measurement not affected by noise and/or errors due to not perfectly rigid coupling between the soft tissues of the limb and the wearable robot.

The control process is therefore more stable and highly performing as it does not have as a reference a non-dimensional gesture value (such as a phase of the rhythmic gesture) but an effectively measured value that describes the trajectory of the articulation.

The present invention has been described with reference to a preferred embodiment thereof. It is to be understood that there may be other embodiments that relate to the same inventive nucleus, all falling within the scope of protection of the claims provided below.

### References

*[1]* Ronsse R., Lenzi T., Vitiello N., Koopman B., van Asseldonk E., De Rossi S.M.M., van den Kieboom J., van derKooij H., Carrozza M.C., Ijspeert A.J., " Oscillator-based assistance of cyclical movements: model-based and model-free approaches" Medical & biological engineering & computing 49.10 (2011): 1173-1185*.*
*[2]* Giovacchini F., Vannetti F., Fantozzi M., Cempini M., Cortese M., Parri A., Yan T., Lefeber D., Vitiello N., "A light-weight active orthosis for hip movement assistance" Robotics and Autonomous Systems 73 (2015): 123-134*.*
*[3]* d'Elia N., Vanetti F., Cempini M., Pasquini G., Parri A., Rabuffetti M., Ferrarin M., Molino Lova R, Vitiello N. " Physical human-robot interaction of an active pelvis orthosis: toward ergonomic assessment of wearable robots. " Journal of neuroengineering and rehabilitation, 14(1), 29, 2017*.*
*[4]* Winter A., "The Biomechanics and Motor Control of Human Gait: Normal, Elderly and Pathological", Waterloo Biomechanics, Fourth edition, 2009*.*

## Claims

1. A wearable robot (1) to be worn by a user comprising:
- at least one motorized module (10) adapted to be arranged at a limb of said user, said module (10) comprising a mobile joint (100) adapted to be associated to an articulation (20) of said limb, said mobile joint being mobile according to at least one degree of freedom associated with a movement of said articulation;
said robotic device module comprising a first kinematic chain comprising, in addition to said mobile joint:
- actuation means (11) for driving or supporting the movement of said joint (100) according to said at least one degree of freedom;
- transmission means (12) interposed between said actuating means and said joint (100);
said robotic device further comprising:
- energy storage and release means, suitable for the activation of said actuation means;
- sensor means (3, 3') suitable for detecting values of movement of the user;
- a control unit (14) designed to control said first kinematic chain of said at least one module as a function of said values detected by said sensor means (3, 3');
**characterized in that**:
said sensor means (3, 3') are arranged at said articulation and are suitable for detecting a detected value of said at least one parameter of said respective movement of said articulation associated with said at least one degree of freedom of said joint, said sensor means (3, 3') being also kinematically uncoupled from said first kinematic chain;
- said control unit (14) is designed to control said first kinematic chain of said at least one module as a function of said value detected by said sensor means (3, 3') of the same module of said at least one parameter of said respective movement of said articulation.

2. The wearable robot according to claim 1, wherein said sensor means (3) comprise elastic means (31, 31') to be worn by said user, adapted to be affected by a deformation as consequence of a movement involving said articulation and also to emit a signal as a function of said deformation, said sensor means being adapted to process said signal in order to obtain said value of said at least one movement parameter of said articulation.

3. The wearable robot according to claim 2, wherein said wearable elastic means are adapted to be affected by a deformation according to a single deformation direction.

4. The wearable robot according to claim 3, wherein said wearable elastic means (31, 31') are adapted to be worn so that said single deformation direction corresponds at least partially to an elongation direction of at least one limb of said user.

5. The wearable robot according to any of the claims from 2 to 4, wherein said wearable elastic means comprise a band of an elasticized and electrically conductive fabric, said signal being obtained as a variation of conductivity proportional to said deformation.

6. The wearable robot according to claim 5, wherein said wearable elastic means comprise two bands of an elasticized and electrically conductive fabric (31, 31') adapted to emit signals as a function respectively of a flexion and extension movement of said at least one limb of said user.

7. The wearable robot according to claim 6, wherein said value of said at least one movement parameter of said articulation is obtained by said deformation signals of two bands adapted to be placed in association with respective portions of said limb involving said articulation, opposite each other according to a plane of flexion-extension of the articulation itself.

8. The wearable robot according to claim 1, wherein said sensor means (3') comprise:
- a sensor (3') adapted to detect the value of said movement parameter on said articulation of said limb;
- a second kinematic chain (4) that connects such sensor to said control unit, said second kinematic chain being uncoupled from said first kinematic chain.

9. The wearable robot according to claim 8, wherein said sensor means comprise an angular sensor, said reference parameter of said movement being an angle of rotation of said articulation.

10. The wearable robot according to claim 9, wherein said sensor is an optical encoder.

11. The wearable robot according to claim 9, wherein said angular sensor is a electrogoniometer.

12. The wearable robot according to claim 9, wherein said angular sensor is a magnetic sensor, such as a Hall effect angular position sensor.

13. The wearable robot according to any one of the previous claims, wherein said motorized module is a rotoidal joint.

## Patentansprüche

1. Tragbarer Roboter (1), der von einem Benutzer getragen wird, enthaltend:
- mindestens ein motorisiertes Modul (10), das ausgelegt ist, um an einem Glied des Benutzers angeordnet zu werden, wobei das Modul (10) ein bewegliches Gelenk (100) enthält, das ausgelegt ist, um mit einem Gelenk (20) des Gliedes verbunden zu werden, wobei das bewegliche Gelenk gemäß mindestens einem Freiheitsgrad beweglich ist, der mit einer Bewegung des Gelenks verbunden ist;
wobei das Robotergerätemodul eine erste kinematische Kette enthält, die zusätzlich zu dem beweglichen Gelenk Folgendes enthält:
- Betätigungsmittel (11) zum Antreiben oder Unterstützen der Bewegung des Gelenks (100) gemäß dem mindestens einen Freiheitsgrad;
- Übertragungsmittel (12), die zwischen den Betätigungsmitteln und dem Gelenk (100) angeordnet sind;
wobei das robotische Vorrichtung weiterhin enthält:
- Energiespeicher- und -abgabemittel, geeignet für die Aktivierung der Betätigungsmittel;
- Sensormittel (3, 3'), die zum Detektieren von Werten der Bewegung des Benutzers geeignet sind;
- eine Steuereinheit (14), die gestaltet ist, die erste kinematische Kette des mindestens einen Moduls als einen Funktion der von den Sensormitteln (3, 3') detektierten Werte zu steuern;
**dadurch gekennzeichnet,**
**dass** die Sensormittel (3, 3') an dem Gelenk angeordnet sind und dazu geeignet sind, einen detektierten Wert des mindestens einen Parameters der jeweiligen Bewegung des Gelenks in Verbindung mit dem mindestens einen Freiheitsgrad des Gelenks zu detektieren, wobei die Sensormittel (3, 3') auch von der ersten kinematischen Kette entkoppelt sind;
- **dass** die Steuereinheit (14) gestaltet ist, um die erste kinematische Kette des mindestens einen Moduls als eine Funktion des Wertes zu steuern, der von den Sensormitteln (3, 3') von dem mindestens einen Parameter der entsprechenden Bewegung des Gelenks detektiert wurde.

2. Tragbarer Roboter nach Anspruch 1, wobei die Sensormittel (3) vom Benutzer getragene elastische Mittel (31, 31') enthalten, die ausgelegt sind, durch eine Verformung als Folge einer Bewegung, die das Gelenk mit einbezieht, beeinflusst zu werden und auch ein Signal als Funktion der genannten Verformung zu emittieren, wobei die Sensormittel ausgelegt sind, das Signal zu verarbeiten, um den Wert des mindestens einen Bewegungsparameters des Gelenks zu erhalten.

3. Tragbarer Roboter nach Anspruch 2, wobei die tragbaren elastischen Mittel dafür ausgelegt sind, durch eine Verformung entsprechend einer einzelnen Verformungsrichtung beeinflusst zu werden.

4. Tragbarer Roboter nach Anspruch 3, wobei die tragbaren elastischen Mittel (31, 31') ausgelegt sind, so dass die einzelne Verformungsrichtung zumindest teilweise einer Streckungsrichtung von mindestens einem Glied des Benutzers entspricht.

5. Tragbarer Roboter nach einem der Ansprüche 2 bis 4, wobei die tragbaren elastischen Mittel ein Band aus einem elastischen und elektrisch leitenden Stoff enthalten, wobei das Signal als eine Änderung der Leitfähigkeit proportional zur Verformung erhalten wird.

6. Tragbarer Roboter nach Anspruch 5, wobei die tragbaren elastischen Mittel zwei Bänder aus einem elastischen und elektrisch leitfähigen Stoff (31, 31') enthalten, die dazu ausgelegt sind, Signale in Abhängigkeit von einer Beugungs- bzw. Streckbewegung des mindestens einen Gliedes des Benutzers zu emittieren.

7. Tragbarer Roboter nach Anspruch 6, wobei der Wert des mindestens einen Bewegungsparameters des Gelenks durch die Verformungssignale von zwei Bändern erhalten wird, die dazu ausgelegt sind, in Verbindung mit jeweiligen Abschnitten des Gliedes, an denen das Gelenk beteiligt ist, einander gegenüberliegend entsprechend einer Beugungs-Streckungsebene des Gelenks selbst platziert zu werden.

8. Tragbarer Roboter nach Anspruch 1, wobei die Sensormittel (3') enthalten:
- einen Sensor (3'), der dazu ausgelegt ist, den Wert des Bewegungsparameters an dem Gelenk des Gliedes zu erfassen;
- ine zweite kinematische Kette (4), die einen solchen Sensor mit der Steuereinheit verbindet, wobei die zweite kinematische Kette von der ersten kinematischen Kette entkoppelt ist.

9. Tragbarer Roboter nach Anspruch 8, wobei die Sensormittel einen Winkelsensor enthalten, wobei der Referenzparameter der Bewegung ein Drehwinkel des Gelenks ist.

10. Tragbarer Roboter nach Anspruch 9, wobei der Sensor ein optischer Encoder ist.

11. Tragbarer Roboter nach Anspruch 9, wobei der Winkelsensor ein Elektrogoniometer ist.

12. Tragbarer Roboter nach Anspruch 9, wobei der Winkelsensor ein magnetischer Sensor ist, beispielsweise ein Hall-Effekt-Winkelpositionssensor.

13. Vorrichtung nach einem der vorhergehenden Ansprüche, wobei das motorisierte Modul ein Rotoidgelenk ist.

## Revendications

1. Un robot portable (1) destiné à être porté par un utilisateur, comprenant :
- au moins un module motorisé (10) adapté pour être disposé au niveau d'un membre dudit utilisateur, ledit module (10) comprenant un joint mobile articulaire (100) adapté pour être associé à une articulation (20) dudit membre, ledit joint mobile étant mobile selon au moins un degré de liberté associé à un mouvement de ladite articulation ;
ledit module du dispositif robotique comprenant une première chaîne cinématique comprenant, en plus dudit joint mobile :
- des moyens d'actionnement (11) pour entraîner ou aider le mouvement dudit joint (100) selon ledit au moins un degré de liberté ;
- des moyens de transmission (12) interposés entre lesdits moyens d'actionnement et ledit joint (100) ;
ledit dispositif robotique comprenant en outre :
- des moyens de stockage et de libération d'énergie, adaptés à l'activation desdits moyens d'actionnement ;
- des moyens capteurs (3, 3') aptes à détecter des valeurs de mouvement de l'utilisateur ;
- une unité de commande (14) agencée pour commander ladite première chaîne cinématique dudit au moins un module selon une fonction desdites valeurs détectées ;
**caractérisé en ce que** :
lesdits moyens capteurs (3, 3') sont montés en coopération avec ladite articulation et sont aptes à détecter une valeur détectée dudit au moins un paramètre dudit mouvement respectif de ladite articulation associé audit au moins un degré de liberté dudit joint, lesdits moyens capteurs (3, 3') étant également cinématiquement découplés de ladite première chaîne cinématique ;
- ladite unité de contrôle (14) est agencée pour commander ladite première chaîne cinématique du au moins un module selon une fonction de ladite valeur détectée par lesdits moyens capteurs (3, 3') du même module dudit au moins un paramètre dudit mouvement respectif de ladite articulation.

2. Le robot portable selon la revendication 1, dans lequel lesdits moyens capteurs (3) comprennent des moyens élastiques (31, 31') pour être portés par ledit utilisateur, adaptés pour être affectés par une déformation à la suite d'un mouvement impliquant ladite articulation et aussi pour émettre une signal en fonction de ladite déformation, lesdits moyens capteurs étant adaptés pour traiter ledit signal afin d'obtenir ladite valeur dudit au moins un paramètre de mouvement de ladite articulation.

3. Le robot portable selon la revendication 2, dans lequel lesdits moyens élastiques portables sont adaptés pour être affectés par une déformation selon une seule direction de déformation.

4. Le robot portable selon la revendication 3, dans lequel lesdits moyens élastiques portables (31, 31') sont adaptés pour être portés de sorte que ladite unique direction de déformation corresponde au moins partiellement à une direction d'allongement d'au moins un membre dudit utilisateur.

5. Le robot portable selon l'une quelconque des revendications 2 à 4, dans lequel lesdits moyens élastiques portables comprennent une bande d'un tissu élastique et électriquement conducteur, ledit signal étant obtenu selon une variation de conductivité proportionnelle à ladite déformation.

6. Le robot portable selon la revendication 5, dans lequel lesdits moyens élastiques portables comprennent deux bandes d'un tissu élastique et électriquement conducteur (31, 31') adaptées pour émettre des signaux en fonction respectivement d'un mouvement de flexion et d'extension dudit au moins un membre dudit utilisateur.

7. Le robot portable selon la revendication 6, dans lequel ladite valeur dudit au moins un paramètre de mouvement de ladite articulation est obtenue par lesdits signaux de déformation des deux bandes adaptées pour être mises en association avec des parties respectives dudit membre impliquant ladite articulation, opposées l'une à l'autre selon un plan de flexion-extension de l'articulation elle-même.

8. Le robot portable selon la revendication 1, dans lequel lesdits moyens capteurs (3') comprennent :
- un capteur (3') adapté pour détecter la valeur dudit paramètre de mouvement sur ladite articulation dudit membre ;
- une seconde chaîne cinématique (4) qui relie ledit capteur à ladite unité de commande, ladite seconde chaîne cinématique étant découplée de ladite première chaîne cinématique.

9. Le robot portable selon la revendication 8, dans lequel lesdits moyens capteurs comprennent un capteur angulaire, ledit paramètre de référence dudit mouvement étant un angle de rotation de ladite articulation.

10. Le robot portable selon la revendication 9, dans lequel ledit capteur est un codeur optique.

11. Le robot portable selon la revendication 9, dans lequel ledit capteur angulaire est un électrogoniomètre.

12. Le robot portable selon la revendication 9, dans lequel ledit capteur angulaire est un capteur magnétique, tel qu'un capteur de position angulaire à effet Hall.

13. Le robot portable selon l'une quelconque des revendications précédentes, dans lequel ledit module motorisé est un joint rotoïdal.
